(19) Europäisches Patentamt
European Patent Office
Office européen des brevets

(11) EP 2 829 271 A2

(12) # EUROPEAN PATENT APPLICATION

(43) Date of publication:
**28.01.2015 Bulletin 2015/05**

(21) Application number: **14183168.5**

(22) Date of filing: **16.04.2010**

(51) Int Cl.:
***A61K 31/382*** *(2006.01)* ***A61K 31/155*** *(2006.01)*
***A61K 31/4439*** *(2006.01)* ***A61K 31/64*** *(2006.01)*
***A61K 45/00*** *(2006.01)* ***A61P 3/10*** *(2006.01)*
***A61P 5/50*** *(2006.01)* ***A61P 9/10*** *(2006.01)*
***A61P 9/14*** *(2006.01)* ***A61P 13/12*** *(2006.01)*
***A61P 25/00*** *(2006.01)* ***A61P 27/02*** *(2006.01)*
***A61P 43/00*** *(2006.01)* ***C07D 335/02*** *(2006.01)*

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **16.04.2009 JP 2009100210**

(62) Document number(s) of the earlier application(s) in accordance with Art. 76 EPC:
**13157571.4 / 2 601 949**
**10764570.7 / 2 419 097**

(71) Applicant: **Taisho Pharmaceutical Co., Ltd.**
**Tokyo 170-8633 (JP)**

(72) Inventors:
- **Takahashi, Teisuke**
  **Tokyo, 170-8633 (JP)**
- **Uchida, Saeko**
  **Tokyo, 170-8633 (JP)**

(74) Representative: **Hoffmann Eitle**
**Patent- und Rechtsanwälte PartmbB**
**Arabellastraße 30**
**81925 München (DE)**

Remarks:
This application was filed on 02-09-2014 as a divisional application to the application mentioned under INID code 62.

(54) **Pharmaceutical compositions with (1S)-1,5-anhydro-1-[5-(4-ethoxybenzyl)-2-methoxy-4-methylphenyl]-1-thio-D-glucitol and a further antidiabetic agent**

(57) A pharmaceutical composition comprising a combination of (A) (1S)-1,5-anhydro-1-[5-(4-ethoxybenzyl)-2-methoxy-4-methylphenyl]-1-thio-D-glucitol and (B) at least one member of the group consisting ofdipeptidyl peptidase IV inhibitors, insulin sensitizers, insulins, α-glucosidase inhibitors, and GLP-1 mimetics, has superior efficacy in preventing or treating diabetes mellitus, diseases associated with diabetes mellitus, or complications of diabetes mellitus and yet it causes no appreciable side effects.

EP 2 829 271 A2

## Description

### Technical Field

The present invention relates to pharmaceutical compositions useful in the treatment of diabetes mellitus, diseases associated with diabetes mellitus, or complications of diabetes mellitus. More particularly, the present invention relates to pharmaceutical compositions comprising combinations of SGLT2 inhibitors and at least one member of the group consisting of biguanides, insulin secretagogues, insulin sensitizers, insulins, dipeptidyl peptidase IV inhibitors, α-glucosidase inhibitors, and GLP-1 mimetics.

### Background Art

Diabetes mellitus is a group of diseases mainly characterized by chronic hyperglycemia due to insufficiency of insulin action and is accompanied by various metabolic abnormalities. The primary treatment of diabetes mellitus consists of exercise and diet therapy; if these methods are incapable of adequate glycemic control, drug therapy with oral antidiabetic agents is conducted. However, depending on the condition of a diabetic patient, treatment with a single drug sometimes encounters difficulty in achieving good glycemic control, and what is more, the development of side effects prevents the drug from being used in an adequate dose or over an adequate period of time. According to one literature report, a biguanide solely used on patients with type 2 diabetes gave only about 25% probability for the possibility of lowering the value of glycated hemoglobin (HbAlc) to 7% or less (see NPL 1). It is also known that if a sulfonylurea is used alone, side effects such as the development of hypoglycemia and weight gain are not the sole problems but that its action of improving hyperglycemia becomes inadequate in patients whose ability to secrete insulin have been deteriorated by failure of beta-cells with the progress of diabetic condition. It is also known that if an insulin sensitizer is used alone, there occurs the problem of occasional development of such side effects as weight gain, edema, heart failure, and hepatopathy. It is also known that if insulin is used alone, there occur the problems of hypoglycemia and weight gain. It is also known that if an α-glucosidase inhibitor is used alone, there occurs the problem of occasional development of abdominal symptoms as side effects. In addition, when a GLP-1 mimetic is used alone, nausea and vomiting are known as therapeutic problems. Furthermore, it is known that patients with type 2 diabetes, when they are exposed to a hyperglycemic condition for a prolonged period of time, will suffer a deterioration in the function of beta-cells; however, most antidiabetic agents are incapable of adequately suppressing the deterioration in the function of beta-cells even if the blood glucose levels are lowered.

As a means of solving the aforementioned problems involved in the treatment with single drugs, a combination therapy is under review that uses a combination of antidiabetic agents that act by different mechanisms. However, few combinations are capable of diminishing the aforementioned problems with the use of single drugs. For example, it is known that the risk for the development of hypoglycemia, a recognized side effect of sulfonylureas, is further increased if they are used in combination with other antidiabetic agents. In addition, the weight gain recognized from sulfonylureas and insulin sensitizers cannot usually be controlled by using them in combination with other antidiabetic agents. Hence, a novel combination of plural pharmaceutical drugs is in demand that can achieve a good glycemic control and which yet causes no appreciable side effects.

Glucose in the blood is filtered in the glomeruli of each kidney and then reabsorbed as mediated by sodium-dependent glucose cotransporters (SGLTs) located at the beginnings of proximal tubules. SGLT2 inhibitors typified by a 1-thio-D-glucitol compound (see PLT 1) inhibit the activity of SGLT2 having low glucose affinity but high glucose transport capacity, so they exhibit a blood glucose reducing action in various animal models by promoting the excretion of urinary glucose.

It was recently reported that side effects of insulin sensitizers could be reduced by combining them with SGLT2 inhibitors (see PLT 2) but there has been no report of a pharmaceutical composition that combines an SGLT2 inhibitor with an insulin sensitizer in order to provide an enhanced blood glucose lowering action. There is also literature teaching the combined use of an SGLT2 inhibitor and a dipeptidyl peptidase IV inhibitor (see PLT 3 and 4). However, no report has been made of pharmaceutical compositions of the type contemplated by the present invention that comprise combinations of SGLT2 inhibitors with at least one member of the group consisting of biguanides, insulin secretagogues, insulin sensitizers, insulins, dipetidyl peptidase IV inhibitors, α-glucosidase inhibitors, and GLP-1 mimetics.

### Citation List

#### Patent Literature

PLT 1: Official Gazette of International Publication WO 2006/073197 A1
PLT 2: Patent Official Gazette of EP 1381361 B1
PLT 3: Official Gazette of International Publication WO 2009/022007 A1
PLT 4: Official Gazette of International Publication WO 2009/022010 A1

Non Patent Literature

NPL 1: Tonyobyougaku (Kiso to Rinshou) [Diabetology (Fundamentals and Clinical Applications)], pp.949-954, 2007, Nishimura Shoten

Summary of Invention

An object of the present invention is to provide a novel pharmaceutical composition comprising a combination of plural drugs that has superior efficacy in preventing or treating diabetes mellitus, diseases associated with diabetes mellitus, or complications of diabetes mellitus and which yet causes no appreciable side effects.
Another object of the present invention is to provide a method of preventing or treating diabetes mellitus, diseases associated with diabetes mellitus, or complications of diabetes mellitus using the pharmaceutical composition.
The present inventors found that when a 1-thio-D-glucitol compound having an SGLT2 inhibiting action or a pharmaceutically acceptable salt thereof or a hydrate of the compound or the salt was administered in combination with at least one member of the group consisting of biguanides, insulin secretagogues, insulin sensitizers, insulins, dipeptidyl peptidase IV inhibitors, α-glucosidase inhibitors, and GLP-1 mimetics, a marked blood glucose lowering action, suppression of the failure of pancreatic β-cells, and alleviation of side effects were caused; the present invention has been accomplished on the basis of this finding.
Thus, the present invention provides the following:

(1) A pharmaceutical composition comprising a combination of

(A) a 1-thio-D-glucitol compound represented by the general formula (I)

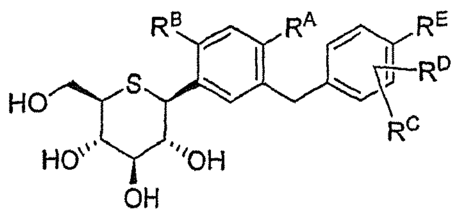

(I)

[where $R^A$ is a hydrogen atom, a $C_{1-6}$ alkyl group, $-OR^F$, or a halogen atom;
$R^B$ is a hydrogen atom, a hydroxyl group, or $-OR^F$;
$R^C$ and $R^D$ which may be the same or different are each a hydrogen atom, a halogen atom, a $C_{1-8}$ alkyl group, or $-OR^F$;
$R^E$ is (i) a hydrogen atom, (ii) a halogen atom, (iii) a hydroxyl group, (iv) a $C_{1-8}$ alkyl group optionally substituted by a halogen atom(s), (v) $-OR^F$, or (vi) $-SR^F$;
$R^F$ is a $C_{1-6}$ alkyl group optionally substituted by a halogen atom(s)] or a pharmaceutically acceptable salt thereof or a hydrate of the compound or the salt; and

(B) at least one member of the group consisting of biguanides, insulin secretagogues, insulin sensitizers, insulins, dipeptidyl peptidase IV inhibitors, α-glucosidase inhibitors, and GLP-1 mimetics.

(2) The pharmaceutical composition according to (1), which comprises a combination of

(A) a 1-thio-D-glucitol compound represented by the general formula (I)

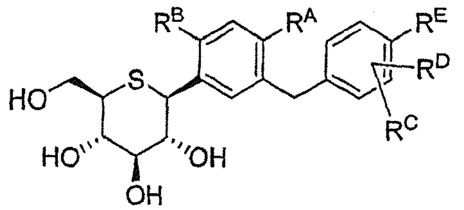

(I)

[where $R^A$ is a hydrogen atom, a $C_{1-6}$ alkyl group, $-OR^F$, or a halogen atom;
$R^B$ is a hydrogen atom, a hydroxyl group, or $-OR^F$;
$R^C$ and $R^D$ which may be the same or different are each a hydrogen atom, a halogen atom, a $C_{1-8}$ alkyl group, or $-OR^F$;
$R^E$ is (i) a hydrogen atom, (ii) a halogen atom, (iii) a hydroxyl group, (iv) a $C_{1-8}$ alkyl group optionally substituted by a halogen atom(s), (v) $-OR^F$, or (vi) $-SR^F$;
$R^F$ is a $C_{1-6}$ alkyl group optionally substituted by a halogen atom(s)] or a pharmaceutically acceptable salt thereof or a hydrate of the compound or the salt; and

(B) a biguanide, an insulin secretagogue, or an insulin sensitizer.

(3) The pharmaceutical composition according to (1) or (2), wherein (A) is the 1-thio-D-glucitol compound represented by the general formula (I)

where $R^A$ is a $C_{1-6}$ alkyl group or a halogen atom;
$R^B$ is a hydrogen atom, a hydroxyl group, or a $C_{1-6}$ alkoxy group;
$R^C$ and $R^D$ are each a hydrogen atom;
$R^E$ is a $C_{1-6}$ alkyl group, a $C_{1-6}$ alkoxy group or a $C_{1-6}$ alkylthio group.

(4) The pharmaceutical composition according to any one of (1) to (3), wherein the 1-thio-D-glucitol compound is selected from the group consisting of:

(1S)-1,5-anhydro-1-[5-(4-ethoxybenzyl)-2-methoxy-4-methylphenyl]-1-thio-D-glucitol;
(1S)-1,5-anhydro-1-[4-chloro-3-(4-methylbenzyl)phenyl]-1-thio-D-glucitol;
(1S)-1,5-anhydro-1-[4-chloro-3-[4-(methylsulfanyl)benzyl]phenyl]-1-thio-D-glucitol;
(1S)-1,5-anhydro-1-[4-chloro-3-(4-ethylbenzyl)phenyl]-1-thio-D-glucitol;
(1S)-1,5-anhydro-1-[5-(4-ethylbenzyl)-2-methoxy-4-methylphenyl]-1-thio-D-glucitol;
(1S)-1,5-anhydro-1-[4-chloro-3-[4-(propan-2-yl)benzyl]phenyl]-1-thio-D-glucitol;
(1S)-1,5-anhydro-1-[2-methoxy-4-methyl-5-[4-(propan-2-yl)benzyl]phenyl]-1-thio-D-glucitol; and
(1S)-1,5-anhydro-1-[4-chloro-5-(4-ethylbenzyl)-2-methoxyphenyl]-1-thio-D-glucitol.

(5) The pharmaceutical composition according to any one of (1) to (4), wherein the 1-thio-D-glucitol compound is (1S)-1,5-anhydro-1-[5-(4-ethoxybenzyl)-2-methoxy-4-methylphenyl]-1-thio-D-glucitol.
(6) The pharmaceutical composition according to any one of (1) to (5), wherein the biguanide is metformin hydrochloride.
(7) The pharmaceutical composition according to any one of (1) to (5), wherein the insulin secretagogue is glipizide, glibenclamide, or glimepiride.
(8) The pharmaceutical composition according to (7), wherein the insulin secretagogue is glipizide.
(9) The pharmaceutical composition according to any one of (1) to (5), wherein the insulin sensitizer is pioglitazone.
(10) The pharmaceutical composition according to any one of (1) and (3) to (5), wherein the dipeptidyl peptidase IV inhibitor is sitagliptin or vildagliptin.
(11) The pharmaceutical composition according to any one of (1) and (3) to (5), wherein the $\alpha$-glucosidase inhibitor is vogllibose, miglitol, or acarbose.
(12) A method of preventing or treating diabetes mellitus, diseases associated with diabetes mellitus, or complications of diabetes mellitus, which comprises administering to a patient in need, either simultaneously or separately:

(A) a 1-thio-D-glucitol compound represented by the general formula (I)

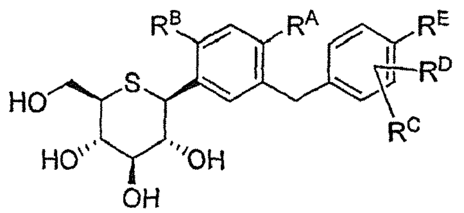

( I )

[where $R^A$ is a hydrogen atom, a $C_{1-6}$ alkyl group, $-OR^F$, or a halogen atom;
$R^B$ is a hydrogen atom, a hydroxyl group, or $-OR^F$;
$R^C$ and $R^D$ which may be the same or different are each a hydrogen atom, a halogen atom, a $C_{1-8}$ alkyl group, or $-OR^F$;
$R^E$ is (i) a hydrogen atom, (ii) a halogen atom, (iii) a hydroxyl group, (iv) a $C_{1-8}$ alkyl group optionally substituted by a halogen atom(s), (v) $-OR^F$, or (vi) $-SR^F$;
$R^F$ is a $C_{1-6}$ alkyl group optionally substituted by a halogen atom(s)] or a pharmaceutically acceptable salt thereof or a hydrate of the compound or the salt; and

(B) at least one member of the group consisting of biguanides, insulin secretagogues, insulin sensitizers, insulins, dipeptidyl peptidase IV inhibitors, $\alpha$-glucosidase inhibitors, and GLP-1 mimetics.

(13) The method of preventing or treating diabetes mellitus, diseases associated with diabetes mellitus, or complications of diabetes mellitus according to (12), which comprises administering to a patient in need, either simultaneously or separately:

(A) a 1-thio-D-glucitol compound represented by the general formula (I)

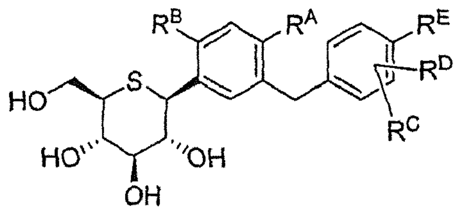

( I )

[where $R^A$ is a hydrogen atom, a $C_{1-6}$ alkyl group, $-OR^F$, or a halogen atom;
$R^B$ is a hydrogen atom, a hydroxyl group, or $-OR^F$;
$R^C$ and $R^D$ which may be the same or different are each a hydrogen atom, a halogen atom, a $C_{1-8}$ alkyl group, or $-OR^F$;
$R^E$ is (i) a hydrogen atom, (ii) a halogen atom, (iii) a hydroxyl group, (iv) a $C_{1-8}$ alkyl group optionally substituted by a halogen atom(s), (v) $-OR^F$, or (vi) $-SR^F$;
$R^F$ is a $C_{1-6}$ alkyl group optionally substituted by a halogen atom(s)] or a pharmaceutically acceptable salt thereof or a hydrate of the compound or the salt; and

(B) a biguanide, an insulin secretagogue, or an insulin sensitizer.

(14) The method of preventing or treating diabetes mellitus, diseases associated with diabetes mellitus, or complications of diabetes mellitus according to (12) or (13), wherein diabetes mellitus is type 2 diabetes.

(15) The method of preventing or treating diabetes mellitus, diseases associated with diabetes mellitus, or complications of diabetes mellitus according to any one of (12) to (14), wherein the complication of diabetes mellitus is diabetic retinopathy, diabetic nephropathy, diabetic neuropathy, cerebrovascular disorder, ischemic cardiac disease, or peripheral arterial disease.

Advantageous Effects of Invention

The pharmaceutical composition of the present invention comprising plural pharmaceutical drugs showed superior glycated hemoglobin (GHb) value lowering action and plasma glucose level lowering action over the individual drugs. In addition, the pharmaceutical composition of the present invention comprising plural pharmaceutical drugs suppressed the lowering of plasma insulin levels that might have occurred with the progress of diabetic condition.

Furthermore, the pharmaceutical composition of the present invention comprising plural pharmaceutical drugs alleviated the side effects of the individual drugs (i.e., weight gain and hypoglycemia).

Description of Embodiments

The "$C_{1-6}$ alkyl group" refers to straight- or branched-chain alkyl groups having 1-6 carbon atoms and may be exemplified by a methyl group, an ethyl group, an n-propyl group, an isopropyl group, an n-butyl group, an isobutyl group, a tert-butyl group, a sec-butyl group, an n-pentyl group, a tert-amyl group, a 3-methylbutyl group, a neopentyl group, and an n-hexyl group.

The "halogen atom" may be exemplified by a fluorine atom, a chlorine atom, a bromine atom, and an iodine atom.

The "pharmaceutically acceptable salt" may be exemplified by: salts with inorganic acids such as sulfuric acid, hydrochloric acid, hydrobromic acid, phosphoric acid, and nitric acid; salts with organic acids such as acetic acid, oxalic acid, lactic acid, tartaric acid, fumaric acid, maleic acid, citric acid, benzenesulofnic acid, methanesulfonic acid, p-toluenesulfonic acid, benzoic acid, camphorsulfonic acid, ethanesulfonic acid, glucoheptonic acid, gluconic acid, glutamic acid, glycolic acid, malic acid, malonic acid, mandelic acid, galactaric acid, and naphthalene-2-sulfonic acid; salts with one or more metal ions such as lithium ion, sodium ion, potassium ion, calcium ion, magnesium ion, zinc ion, and aluminum ion; and salts with amines such as ammonia, arginine, lysine, piperazine, choline, diethylamine, 4-phenylcyclohexylamine, 2-aminoethanol, and benzathine.

The compounds of the present invention can exist as various solvates. They may also be hydrates in view of their applicability as pharmaceutical drugs.

The compounds of the present invention encompass all cases including enantiomers, diastereomers, equilibrium compounds, mixtures of these forms in desired proportions, and racemic bodies.

The methods of producing the 1-thio-D-glucitol compounds of formula (I) which are used in the present invention, as well as the pharmaceutically acceptable salts thereof and the hydrates of the compounds or salts which are also used in the present invention are disclosed in the official gazette of International Publication WO 2006/073197 A1.

Among the 1-thio-D-glucitol compounds of formula (I), the following are preferred because they exhibit a superior SGLT2 inhibiting activity:

(1S)-1,5-anhydro-1-[5-(4-ethoxybenzyl)-2-methoxy-4-methylphenyl]-1-thio-D-glucitol (formula (IA)),

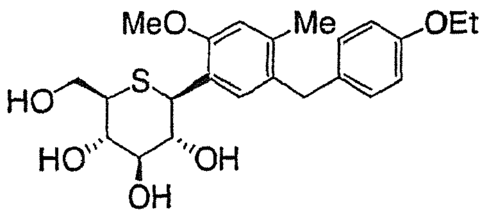

(IA)

(1S)-1,5-anhydro-1-[4-chloro-3-(4-methylbenzyl)phenyl]-1-thio-D-glucitol (formula (IB)),

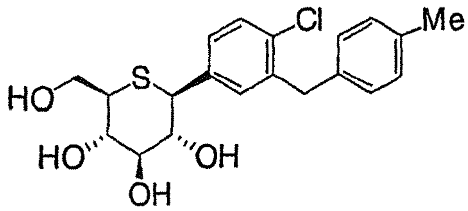

(IB)

(1S)-1,5-anhydro-1-[4-chloro-3-[4-(methylsulfanyl)benzyl]phenyl]-1-thio-D-glucitol (formula (IC)),

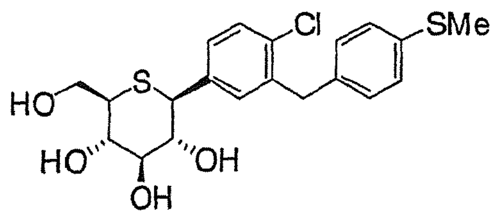

(IC)

(1S)-1,5-anhydro-1-[4-chloro-3-(4-ethylbenzyl)phenyl]-1-thio-D-glucitol (formula (ID)),

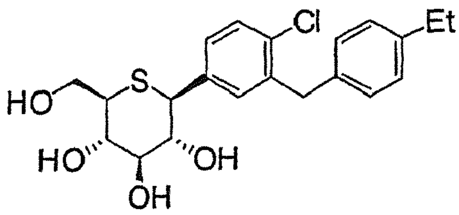

(ID)

(1S)-1,5-anhydro-1-[5-(4-ethylbenzyl)-2-methoxy-4-methylphenyl]-1-thio-D-glucitol (formula (IE)),

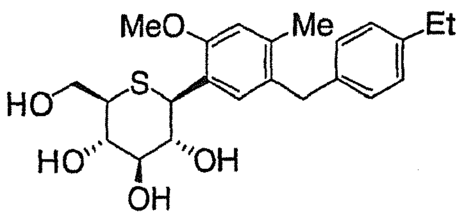

(IE)

(1S)-1,5-anhydro-1-[4-chloro-3-[4-(propan-2-yl)benzyl]phenyl]-1-thio-D-glucitol (formula (IF)),

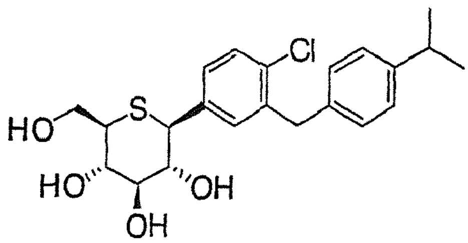

(IF)

(1S)-1,5-anhydro-1-[2-methoxy-4-methyl-5-[4-(propan-2-yl)benzyl]phenyl]-1-thio-D-glucitol (formula (IG)),

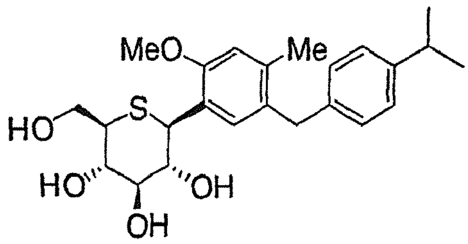

(IG)

(1S)-1,5-anhydro-1-[4-chloro-5-(4-ethylbenzy1)-2-methoxyphenyl]-1-thio-D-glucitol (formula (IH)).

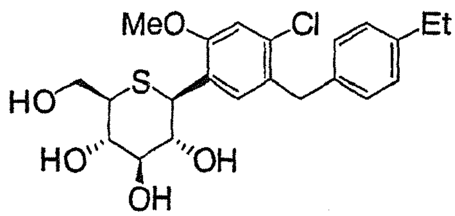

(IH)

A more preferred compound is (1S)-1,5-anhydro-1-[5-(4-ethoxybenzyl)-2-methoxy-4-methylphenyl]-1-thio-D-glucitol, which is preferably a hydrate.

One mode of the present invention is a pharmaceutical composition comprising the 1-thio-D-glucitol compound of formula (I) or a pharmaceutically acceptable salt thereof or a hydrate of the compound or salt and a biguanide. This pharmaceutical composition is preferably a medicine against diabetes mellitus, diseases associated with diabetes mellitus, or complications of diabetes mellitus.

Another mode is a method of preventing or treating diabetes mellitus, diseases associated with diabetes mellitus, or complications of diabetes mellitus, which comprises administering a patient in need the 1-thio-D-glucitol compound of formula (I) or a pharmaceutically acceptable salt thereof or a hydrate of the compound or salt and a biguanide, either simultaneously or separately.

Biguanides are drugs having actions of suppression of hepatic gluconeogenesis, increase of insulin sensitivity in the peripheral tissues, inhibition of glucose absorption from the intestine. Exemplary biguanides are metformin and buformin. Metformin is preferred from the viewpoints of blood glucose lowering action, limited side effects, and the like, with metformin hydrochloride being particularly preferred. These biguanides are known substances and metformin and metformin hydrochloride in particular are disclosed in Emil A. Werner and James Bell, J. Chem. Soc., 121, 1922, 1790-1794, and a commercial product having the trademark GLUCOPHAGE may be used.

Still another mode of the present invention is a pharmaceutical composition comprising the 1-thio-D-glucitol compound of formula (I) or a pharmaceutically acceptable salt thereof or a hydrate of the compound or salt and an insulin secretagogue. This pharmaceutical composition is preferably a medicine against diabetes mellitus, diseases associated with diabetes mellitus, or complications of diabetes mellitus.

Yet another mode is a method of preventing or treating diabetes mellitus, diseases associated with diabetes mellitus, or complications of diabetes mellitus, which comprises administering a patient in need the 1-thio-D-glucitol compound of formula (I) or a pharmaceutically acceptable salt thereof or a hydrate of the compound or salt and an insulin secretagogue, either simultaneously or separately.

Insulin secretagogues are drugs having the property to promote insulin secretion from pancreatic β-cells. Exemplary insulin secretagogues are sulfonylureas (glipizide, glibenclamide, glimepiride, gliclazide, acetohexamide, tolbutamide, glyclopyramide, chlorpropamide, and tolazamide) and glinides (nateglinide, mitiglinide, and repaglinide). Among these, glipizide, glibenclamide and glimepiride which are sulfonylureas are particularly preferred from the viewpoint of blood glucose lowering action. These insulin secretagogues are known substances and commercial products may typically be used.

Still another mode of the present invention is a pharmaceutical composition comprising the 1-thio-D-glucitol compound of formula (I) or a pharmaceutically acceptable salt thereof or a hydrate of the compound or salt and an insulin sensitizer. This pharmaceutical composition is preferably a medicine against diabetes mellitus, diseases associated with diabetes mellitus, or complications of diabetes mellitus.

Yet another mode is a method of preventing or treating diabetes mellitus, diseases associated with diabetes

mellitus, or complications of diabetes mellitus, which comprises administering a patient in need the 1-thio-D-glucitol compound of formula (I) or a pharmaceutically acceptable salt thereof or a hydrate of the compound or salt and an insulin sensitizer, either simultaneously or separately.

Insulin sensitizers are drugs which improve insulin sensitivity in the peripheral tissues and the liver. Exemplary insulin sensitizers are pioglitazone and rosiglitazone. Pioglitazone is preferred from the viewpoints of blood glucose lowering action, limited side effects, and the like, with pioglitazone hydrochloride being particularly preferred. These insulin sensitizers are known substances and commercial products may typically be used.

Still another mode of the present invention is a pharmaceutical composition comprising the 1-thio-D-glucitol compound of formula (I) or a pharmaceutically acceptable salt thereof or a hydrate of the compound or salt and an insulin. This pharmaceutical composition is preferably a medicine against diabetes mellitus, diseases associated with diabetes mellitus, or complications of diabetes mellitus.

Yet another mode is a method of preventing or treating diabetes mellitus, diseases associated with diabetes mellitus, or complications of diabetes mellitus, which comprises administering a patient in need the 1-thio-D-glucitol compound of formula (I) or a pharmaceutically acceptable salt thereof or a hydrate of the compound or salt and an insulin, either simultaneously or separately.

Preferred exemplary insulins are human insulin preparations (e.g., human insulin injection as solution, biosynthetic human neutral insulin injection as solution, human isophane insulin injection as aqueous suspension, biosynthetic human isophane insulin injection as aqueous suspension, biosynthetic human biphasic isophane insulin injection as aqueous suspension, and human insulin injection as aqueous suspension) and insulin analog preparations (insulin lispro, insulin aspart, insulin glulisine, insulin glargine, and insulin detemir), with human insulin preparations being particularly preferred. Although insulins include various types such as ultra-fast-acting types, fast-acting types, biphasic types, intermediate types and sustained types, these can be selected and administered according to the patients's condition. These insulin preparations are known substances and commercial products may typically be used.

Still another mode of the present invention is a pharmaceutical composition comprising the 1-thio-D-glucitol compound of formula (I) or a pharmaceutically acceptable salt thereof or a hydrate of the compound or salt and a dipeptidyl peptidase IV inhibitor. This pharmaceutical composition is preferably a medicine against diabetes mellitus, diseases associated with diabetes mellitus, or complications of diabetes mellitus.

Yet another mode is a method of preventing or treating diabetes mellitus, diseases associated with diabetes mellitus, or complications of diabetes mellitus, which comprises administering a patient in need the 1-thio-D-glucitol compound of formula (I) or a pharmaceutically acceptable salt thereof or a hydrate of the compound or salt and a dipeptidyl peptidase IV inhibitor, either simultaneously or separately.

Dipeptidyl peptidase IV enzyme inactivates the incretin hormones glucagons like peptide-1 (GLP-1) and glucose-dependent insulinotropic polypeptide (GIP). GLP-1 and GIP play a key role in the regulation of insulin secretion and glucose homeostasis. Dipeptidyl peptidase IV inhibitors are drugs which prevent inactivation of these peptides and enhance glucose-dependent insulin secretion from pancreatic $\beta$ cells. Preferred exemplary dipeptidyl peptidase IV inhibitors are sitagliptin, vildagliptin, saxagliptin, alogliptin, linagliptin, teneligliptin, SK-0403, carmegliptin, KRP-104, and SYR-472, with sitagliptin and vildagliptin being particularly preferred. These dipeptidyl peptidase IV inhibitors are known substances and commercial products may typically be used.

Still another mode of the present invention is a pharmaceutical composition comprising the 1-thio-D-glucitol compound of formula (I) or a pharmaceutically acceptable salt thereof or a hydrate of the compound or salt and an $\alpha$-glucosidase inhibitor. This pharmaceutical composition is preferably a medicine against diabetes mellitus, diseases associated with diabetes mellitus, or complications of diabetes mellitus.

Yet another mode is a method of preventing or treating diabetes mellitus, diseases associated with diabetes mellitus, or complications of diabetes mellitus, which comprises administering a patient in need the 1-thio-D-glucitol compounds of formula (I) or a pharmaceutically acceptable salt thereof or a hydrate of the compound or salt and an $\alpha$-glucosidase inhibitor, either simultaneously or separately.

$\alpha$-Glucosidase inhibitors are drugs which inhibit digestive enzymes such as amylase, maltase, $\alpha$-dextrinase, sucrase, etc. to delay carbohydrate absorption from the small intestine. Preferred exemplary $\alpha$-glucosidase inhibitors are voglibose, miglitol, and acarbose. These $\alpha$-glucosidase inhibitors are known substances and commercial products may typically be used.

Still another mode of the present invention is a pharmaceutical composition comprising the 1-thio-D-glucitol compound of formula (I) or a pharmaceutically acceptable salt thereof or a hydrate of the compound or salt and a GLP-1 mimetic. This pharmaceutical composition is preferably a medicine against diabetes mellitus, diseases associated with diabetes mellitus, or complications of diabetes mellitus.

Yet another mode is a method of preventing or treating diabetes mellitus, diseases associated with diabetes mellitus, or complications of diabetes mellitus, which comprises administering a patient in need the 1-thio-D-glucitol compounds of formula (I) or a pharmaceutically acceptable salt thereof or a hydrate of the compound or salt and a GLP-1 mimetic, either simultaneously or separately.

GLP-1 mimetics are drugs having insulinotropic action by activating the human GLP-1 receptor. GLP-1 mimetics are taken to be any compound including peptides and non-peptide compounds. Preferred exemplary GLP-1 mimetics are liraglutide, exenatide, taspoglutide, and albiglutide, with liraglutide being particularly preferred. These GLP-1 mimetics are known substances and commercial products may typically be used.

Still another mode of the present invention is a pharmaceutical composition comprising an Active Ingredient A or a pharmaceutically acceptable salt thereof or a hydrate of the ingredient or salt and an Active Ingredient B as shown in Table A. This pharmaceutical composition is preferably a medicine against diabetes mellitus, diseases associated with diabetes mellitus, or complications of diabetes mellitus.

Yet another mode is a method of preventing or treating diabetes mellitus, diseases associated with diabetes mellitus, or complications of diabetes mellitus, which comprises administering a patient in need an Active Ingredient A or a pharmaceutically acceptable salt thereof or a hydrate of the ingredient or salt and an Active Ingredient B as shown in Table A, either simultaneously or separately.

Table A

| Active Ingredient A | Active Ingredient B |
|---|---|
| Compound of formula (IA) | metformin |
| Compound of formula (IA) | buformin |
| Compound of formula (IA) | glipizide |
| Compound of formula (IA) | glibenclamide |
| Compound of formula (IA) | glimepiride |
| Compound of formula (IA) | gliclazide |
| Compound of formula (IA) | acetohexamide |
| Compound of formula (IA) | tolbutamide |
| Compound of formula (IA) | glyclopyramide |
| Compound of formula (IA) | chlorpropamide |
| Compound of formula (IA) | tolazamide |
| Compound of formula (IA) | nateglinide |
| Compound of formula (IA) | mitiglinide |
| Compound of formula (IA) | repaglinide |
| Compound of formula (IA) | pioglitazone |
| Compound of formula (IA) | rosiglitazone |
| Compound of formula (IA) | human insulin preparations |
| Compound of formula (IA) | insulin analog preparations |
| Compound of formula (IA) | sitagliptin |
| Compound of formula (IA) | vildagliptin |
| Compound of formula (IA) | saxagliptin |
| Compound of formula (IA) | alogliptin |
| Compound of formula (IA) | linagliptin |
| Compound of formula (IA) | teneligliptin |
| Compound of formula (IA) | voglibose |
| Compound of formula (IA) | miglitol |
| Compound of formula (IA) | acarbose |
| Compound of formula (IA) | liraglutide |
| Compound of formula (IA) | exenatide |

(continued)

| Active Ingredient A | Active Ingredient B |
|---|---|
| Compound of formula (IA) | albiglutide |
| Compound of formula (IB) | metformin |
| Compound of formula (IB) | buformin |
| Compound of formula (IB) | glipizide |
| Compound of formula (IB) | glibenclamide |
| Compound of formula (IB) | glimepiride |
| Compound of formula (IB) | gliclazide |
| Compound of formula (IB) | acetohexamide |
| Compound of formula (IB) | tolbutamide |
| Compound of formula (IB) | glyclopyramide |
| Compound of formula (IB) | chlorpropamide |
| Compound of formula (IB) | tolazamide |
| Compound of formula (IB) | nateglinide |
| Compound of formula (IB) | mitiglinide |
| Compound of formula (IB) | repaglinide |
| Compound of formula (IB) | pioglitazone |
| Compound of formula (IB) | rosiglitazone |
| Compound of formula (IB) | human insulin preparations |
| Compound of formula (IB) | insulin analog preparations |
| Compound of formula (IB) | sitagliptin |
| Compound of formula (IB) | vildagliptin |
| Compound of formula (IB) | saxagliptin |
| Compound of formula (IB) | alogliptin |
| Compound of formula (IB) | linagliptin |
| Compound of formula (IB) | teneligliptin |
| Compound of formula (IB) | voglibose |
| Compound of formula (IB) | miglitol |
| Compound of formula (IB) | acarbose |
| Compound of formula (IB) | liraglutide |
| Compound of formula (IB) | exenatide |
| Compound of formula (IB) | albiglutide |
| Compound of formula (IC) | metformin |
| Compound of formula (IC) | buformin |
| Compound of formula (IC) | glipizide |
| Compound of formula (IC) | glibenclamide |
| Compound of formula (IC) | glimepiride |
| Compound of formula (IC) | gliclazide |
| Compound of formula (IC) | acetohexamide |

(continued)

| Active Ingredient A | Active Ingredient B |
|---|---|
| Compound of formula (IC) | tolbutamide |
| Compound of formula (IC) | glyclopyramide |
| Compound of formula (IC) | chlorpropamide |
| Compound of formula (IC) | tolazamide |
| Compound of formula (IC) | nateglinide |
| Compound of formula (IC) | mitiglinide |
| Compound of formula (IC) | repaglinide |
| Compound of formula (IC) | pioglitazone |
| Compound of formula (IC) | rosiglitazone |
| Compound of formula (IC) | human insulin preparations |
| Compound of formula (IC) | insulin analog preparations |
| Compound of formula (IC) | sitagliptin |
| Compound of formula (IC) | vildagliptin |
| Compound of formula (IC) | saxagliptin |
| Compound of formula (IC) | alogliptin |
| Compound of formula (IC) | linagliptin |
| Compound of formula (IC) | teneligliptin |
| Compound of formula (IC) | voglibose |
| Compound of formula (IC) | miglitol |
| Compound of formula (IC) | acarbose |
| Compound of formula (IC) | liraglutide |
| Compound of formula (IC) | exenatide |
| Compound of formula (IC) | albiglutide |
| Compound of formula (ID) | metformin |
| Compound of formula (ID) | buformin |
| Compound of formula (ID) | glipizide |
| Compound of formula (ID) | glibenclamide |
| Compound of formula (ID) | glimepiride |
| Compound of formula (ID) | gliclazide |
| Compound of formula (ID) | acetohexamide |
| Compound of formula (ID) | tolbutamide |
| Compound of formula (ID) | glyclopyramide |
| Compound of formula (ID) | chlorpropamide |
| Compound of formula (ID) | tolazamide |
| Compound of formula (ID) | nateglinide |
| Compound of formula (ID) | mitiglinide |
| Compound of formula (ID) | repaglinide |
| Compound of formula (ID) | pioglitazone |

(continued)

| Active Ingredient A | Active Ingredient B |
|---|---|
| Compound of formula (ID) | rosiglitazone |
| Compound of formula (ID) | human insulin preparations |
| Compound of formula (ID) | insulin analog preparations |
| Compound of formula (ID) | sitagliptin |
| Compound of formula (ID) | vildagliptin |
| Compound of formula (ID) | saxagliptin |
| Compound of formula (ID) | alogliptin |
| Compound of formula (ID) | linagliptin |
| Compound of formula (ID) | teneligliptin |
| Compound of formula (ID) | voglibose |
| Compound of formula (ID) | miglitol |
| Compound of formula (ID) | acarbose |
| Compound of formula (ID) | liraglutide |
| Compound of formula (ID) | exenatide |
| Compound of formula (ID) | albiglutide |
| Compound of formula (IE) | metformin |
| Compound of formula (IE) | buformin |
| Compound of formula (IE) | glipizide |
| Compound of formula (IE) | glibenclamide |
| Compound of formula (IE) | glimepiride |
| Compound of formula (IE) | gliclazide |
| Compound of formula (IE) | acetohexamide |
| Compound of formula (IE) | tolbutamide |
| Compound of formula (IE) | glyclopyramide |
| Compound of formula (IE) | chlorpropamide |
| Compound of formula (IE) | tolazamide |
| Compound of formula (IE) | nateglinide |
| Compound of formula (IE) | mitiglinide |
| Compound of formula (IE) | repaglinide |
| Compound of formula (IE) | pioglitazone |
| Compound of formula (IE) | rosiglitazone |
| Compound of formula (IE) | human insulin preparations |
| Compound of formula (IE) | insulin analog preparations |
| Compound of formula (IE) | sitagliptin |
| Compound of formula (IE) | vildagliptin |
| Compound of formula (IE) | saxagliptin |
| Compound of formula (IE) | alogliptin |
| Compound of formula (IE) | linagliptin |

(continued)

| Active Ingredient A | Active Ingredient B |
|---|---|
| Compound of formula (IE) | teneligliptin |
| Compound of formula (IE) | voglibose |
| Compound of formula (IE) | miglitol |
| Compound of formula (IE) | acarbose |
| Compound of formula (IE) | liraglutide |
| Compound of formula (IE) | exenatide |
| Compound of formula (IE) | albiglutide |
| Compound of formula (IF) | metformin |
| Compound of formula (IF) | buformin |
| Compound of formula (IF) | glipizide |
| Compound of formula (IF) | glibenclamide |
| Compound of formula (IF) | glimepiride |
| Compound of formula (IF) | gliclazide |
| Compound of formula (IF) | acetohexamide |
| Compound of formula (IF) | tolbutamide |
| Compound of formula (IF) | glyclopyramide |
| Compound of formula (IF) | chlorpropamide |
| Compound of formula (IF) | tolazamide |
| Compound of formula (IF) | nateglinide |
| Compound of formula (IF) | mitiglinide |
| Compound of formula (IF) | repaglinide |
| Compound of formula (IF) | pioglitazone |
| Compound of formula (IF) | rosiglitazone |
| Compound of formula (IF) | human insulin preparations |
| Compound of formula (IF) | insulin analog preparations |
| Compound of formula (IF) | sitagliptin |
| Compound of formula (IF) | vildagliptin |
| Compound of formula (IF) | saxagliptin |
| Compound of formula (IF) | alogliptin |
| Compound of formula (IF) | linagliptin |
| Compound of formula (IF) | teneligliptin |
| Compound of formula (IF) | voglibose |
| Compound of formula (IF) | miglitol |
| Compound of formula (IF) | acarbose |
| Compound of formula (IF) | liraglutide |
| Compound of formula (IF) | exenatide |
| Compound of formula (IF) | albiglutide |
| Compound of formula (IG) | metformin |

(continued)

| Active Ingredient A | Active Ingredient B |
|---|---|
| Compound of formula (IG) | buformin |
| Compound of formula (IG) | glipizide |
| Compound of formula (IG) | glibenclamide |
| Compound of formula (IG) | glimepiride |
| Compound of formula (IG) | gliclazide |
| Compound of formula (IG) | acetohexamide |
| Compound of formula (IG) | tolbutamide |
| Compound of formula (IG) | glyclopyramide |
| Compound of formula (IG) | chlorpropamide |
| Compound of formula (IG) | tolazamide |
| Compound of formula (IG) | nateglinide |
| Compound of formula (IG) | mitiglinide |
| Compound of formula (IG) | repaglinide |
| Compound of formula (IG) | pioglitazone |
| Compound of formula (IG) | rosiglitazone |
| Compound of formula (IG) | human insulin preparations |
| Compound of formula (IG) | insulin analog preparations |
| Compound of formula (IG) | sitagliptin |
| Compound of formula (IG) | vildagliptin |
| Compound of formula (IG) | saxagliptin |
| Compound of formula (IG) | alogliptin |
| Compound of formula (IG) | linagliptin |
| Compound of formula (IG) | teneligliptin |
| Compound of formula (IG) | voglibose |
| Compound of formula (IG) | miglitol |
| Compound of formula (IG) | acarbose |
| Compound of formula (IG) | liraglutide |
| Compound of formula (IG) | exenatide |
| Compound of formula (IG) | albiglutide |
| Compound of formula (IH) | metformin |
| Compound of formula (IH) | buformin |
| Compound of formula (IH) | glipizide |
| Compound of formula (IH) | glibenclamide |
| Compound of formula (IH) | glimepiride |
| Compound of formula (IH) | gliclazide |
| Compound of formula (IH) | acetohexamide |
| Compound of formula (IH) | tolbutamide |
| Compound of formula (IH) | glyclopyramide |

(continued)

| Active Ingredient A | Active Ingredient B |
|---|---|
| Compound of formula (IH) | chlorpropamide |
| Compound of formula (IH) | tolazamide |
| Compound of formula (IH) | nateglinide |
| Compound of formula (IH) | mitiglinide |
| Compound of formula (IH) | repaglinide |
| Compound of formula (IH) | pioglitazone |
| Compound of formula (IH) | rosiglitazone |
| Compound of formula (IH) | human insulin preparations |
| Compound of formula (IH) | insulin analog preparations |
| Compound of formula (IH) | sitagliptin |
| Compound of formula (IH) | vildagliptin |
| Compound of formula (IH) | saxagliptin |
| Compound of formula (IH) | alogliptin |
| Compound of formula (IH) | linagliptin |
| Compound of formula (IH) | teneligliptin |
| Compound of formula (IH) | voglibose |
| Compound of formula (IH) | miglitol |
| Compound of formula (IH) | acarbose |
| Compound of formula (IH) | liraglutide |
| Compound of formula (IH) | exenatide |
| Compound of formula (IH) | albiglutide |

"Diabetes mellitus" encompasses type 1 diabetes, type 2 diabetes, and other types of diabetes mellitus due to specific causes. The diseases targeted by the pharmaceutical drugs of the present invention are preferably type 1 diabetes and type 2 diabetes.

"Diseases associated with diabetes mellitus" include obesity, hyperinsulinemia, dysglycemia (glucose metabolism disorder), hyperlipidemia, hypercholesterolemia, hypertriglyceridemia, dyslipidemia (lipid metabolism disorder), hypertension, congestive heart failure, edema, hyperuricemia, and gout.

"Complications of diabetes mellitus" are classified into acute and chronic ones.

"Acute complications" include hyperglycemia (e.g., ketoacidosis), hyperglycemic hyperosmolar syndrome, lactic acidosis, hypoglycemia, and infectious diseases (e.g., skin, soft tissue, biliary, respiratory, and urinary-tract infections).

"Chronic complications" include microangiopathy (diabetic retinopathy, diabetic neuropathy, and diabetic nephropathy), as well as macroangiopathy (cerebrovascular disorder, ischemic heart disease, and peripheral arterial disease).

"Treatment" means administering the pharmaceutical compositions of the present invention to patients who have already manifested diseases such as diabetes mellitus, diseases associated with diabetes mellitus, or complications of diabetes mellitus. Acts of this treatment cover symptomatic therapy intended to mitigate the symptoms derived from the above-mentioned diseases. Also included are treatments for partial or complete recovery from disease, as well as treatments that stop or retard the progress of disease.

"Prevention" means a practice by which patients who have a risk for manifesting diseases such as diabetes mellitus, diseases associated with diabetes mellitus, or complications of diabetes mellitus are administered the pharmaceutical compositions of the present invention before the disease manifests itself.

The pharmaceutical composition of the present invention can be such that the active ingredients described above are formulated as a single preparation (combination preparation) or they are separately formulated as two or more preparations. These preparations may be tablets, granules, powders, capsules, emulsions, suspensions or syrups

or alternatively they may be injections in such forms as a sterile solution or a liquid suspension, all being obtained by commonly employed means. If the active ingredients are separately formulated as two or more preparations, the respective preparations may be administered either simultaneously or at given time intervals spaced apart. The two or more preparations may be administered at different frequencies in a day. The pharmaceutical composition of the present invention may be administered either systemically or topically by an oral or parenteral route. If the active ingredients are separately formulated as two or more preparations, the respective preparations may be administered by different routes.

If the pharmaceutical composition of the present invention is formulated as two different preparations, they are highly likely to be administered either simultaneously or at a very short interval, so a document such as a package insert or a sales brochure that accompanies a commercial product preferably states to the effect that the two preparations should be administered in combination. Another preferred embodiment is a kit of two preparations, one comprising the 1-thio-D-glucitol compound and the other comprising at least one member of the group consisting of biguanides, insulin secretagogues, insulin sensitizers, insulins, dipeptidyl peptidase IV inhibitors, α-glucosidase inhibitors, and GLP-1 mimetics.

The dosage of the pharmaceutical composition of the present invention varies with the target, method, etc. of administration. Take, for example, the case of oral administration; a patient with diabetes mellitus is preferably administered the following daily doses:

(1) 0.1-50 mg, preferably 0.5-25 mg, of the 1-thio-D-glucitol compound;
(2) 10-3000 mg of biguanides;

  (2-1) 100-3000 mg, preferably 300-3000 mg, of metformin;
  (2-2) 10-500 mg, preferably 30-150 mg, of buformin;

(3) 0.5-2000 mg of insulin secretagogues;

  (3-1) 0.5-100 mg, preferably 1-10 mg, of glimepiride;
  (3-2) 0.5-100 mg, preferably 1-10 mg, of glibenclamide;
  (3-3) 10-2000 mg, preferably 100-1000 mg, of acetohexamide;
  (3-4) 100-2000 mg, preferably 300-2000 mg, of tolbutamide;
  (3-5) 50-2000 mg, preferably 100-500 mg, of glyclopyramide;
  (3-6) 10-1000 mg, preferably 50-500 mg, of chlorpropamide;
  (3-7) 0.5-2000 mg, preferably 2-40 mg, of glipizide;
  (3-8) 10-2000 mg, preferably 50-500 mg, of tolazamide;
  (3-9) 10-500 mg, preferably 30-200 mg, of gliclazide;

(4) 1-100 mg of insulin sensitizers;

  (4-1) 1-100 mg, preferably 10-50 mg, of pioglitazone;

(5) 1-3000 units, preferably 10-1000 units, of insulins;
(6) 1-300 mg of dipeptidyl peptidase IV inhibitors;

  (6-1) 1-300 mg, preferably 20-100 mg, of sitagliptin;
  (6-2) 1-300 mg, preferably 20-100 mg, of vildagliptin;

(7) 0.2-1000 mg of α-glucosidase inhibitors;

  (7-1) 0.2-100 mg, preferably 0.5-10 mg, of voglibose;
  (7-2) 10-1000 mg, preferably 100-500 mg, of miglitol; and
  (7-3) 10-1000 mg, preferably 100-500 mg, of acarbose;

for the case of injection; a patient with diabetes mellitus is preferably administered the following daily doses:

(1) 1-100 units, preferably 4-100 units, of insulins;
(2) 0.001-300 mg of GLP-1 mimetics;

  (2-1) 0.1-10 mg, preferably 0.3-3 mg, of liragultide;
  (2-2) 0.001-0.1 mg, preferably 0.005-0.05 mg, of exenatide; and

(2-3) 0.3-300 mg, preferably 1-100 mg, of albiglutide.

Biguanides are usually administered in two or three divided portions. On the other hand, the 1-thio-D-glucitol compound can be rendered to exhibit a sustained SGLT2 inhibiting action for an extended period. Hence, to design a combination preparation of a type that is to be administered once a day, the 1-thio-D-glucitol compound of the present invention is preferably used in combination with "a biguanide designed to be capable of sustained release."

The "biguanide designed to be capable of sustained release" can be prepared in accordance with known methods. For example, biguanides can be rendered to be capable of sustained release by using the slow-releasing method described in the official gazette of WO 96/08243 A1 or the method described in the official gazette of JP 2003-520759 A.

The preparations described above are preferably oral preparations such as tablets, granules, powders, capsules, emulsions, suspensions, and syrups. Specifically, the active ingredients described above may be mixed, either simultaneously or separately, with excipients such as mannitol and lactose and, after granulation, filled into capsules either directly or after being mixed with other additives for oral administration, which are specifically exemplified by excipients (e.g., sugar-based or sugar alcoholic excipients such as glucose, sucrose, mannitol, lactose, xylitol, sorbitol, maltitol, and pullulan; cellulosic excipients such as microcrystalline cellulose; starch-based excipients such as corn starch; inorganic excipients such as anhydrous dibasic calcium phosphate), binders (e.g., cellulosic binders such as methylcellulose, hydroxypropyl cellulose, and hydroxypropyl methylcellulose), disintegrants (e.g., cellulosic disintegrants such as carmellose calcium, low-substitution hydroxypropyl cellulose, and croscarmelose sodium, or starch-based disintegrants such as partially gelatinized starch and carboxymethyl starch sodium), fluidizers (e.g., inorganic fluidizers such as light silicic anhydride), or lubricants (e.g., stearic acid, magnesium stearate, calcium stearate, talc, and sodium stearyl fumarate); alternatively, the granulation may be pelletized into tablets.

The proportion at which the 1-thio-D-glucitol compound of the present invention or a pharmaceutically acceptable salt thereof or a hydrate of the compound or salt is combined with at least one member of the group consisting of biguanides, insulin secretagogues, insulin sensitizers, insulins, dipeptidyl peptidase IV inhibitors, $\alpha$-glucosidase inhibitors, and GLP-1 mimetics varies with the target, method, etc. of administration. Take, for example, the case of administering the pharmaceutical composition of the present invention to a human; if one part by mass of the 1-thio-D-glucitol compound is combined with 0.01-1000 parts by mass of at least one member of the group consisting of biguanides, insulin secretagogues, insulin sensitizers, insulins, dipeptidyl peptidase IV inhibitors, $\alpha$-glucosidase inhibitors, and GLP-1 mimetics, it is possible to obtain a superior blood glucose lowering action than is achieved by administering the individual drugs. It is particularly preferred to combine one part by mass of the 1-thio-D-glucitol compound in combination with 0.1-100 parts by mass of at least one member of the group consisting of biguanides, insulin secretagogues, insulin sensitizers, insulins, dipeptidyl peptidase IV inhibitors, $\alpha$-glucosidase inhibitors, and GLP-1 mimetics. This enables satisfactory efficacy to be obtained by using the respective drugs in smaller amounts than when they are administered individually. As a further advantage, the pharmaceutical composition of the present invention has fewer side effects because it does not cause hypoglycemia or weight gain that might result from excessive insulin secretion.

Patients who are to be treated by the pharmaceutical composition of the present invention are those who are unable to have good glycemic control in spite of continued diet and exercise and who therefore need be subjected to drug therapy; preferred patients are those who are unable to have good glycemic control in spite of being administered with a single oral antidiabetic drug and who therefore need to take another drug that acts by a different mechanism.

The pharmaceutical composition of the present invention can typically be produced according to the following formulas.

(Preparation 1) Tablet

Tablets with a diameter of 13 mm were prepared; each tablet contained:

| | |
|---|---|
| (1S)-1,5-anhydro-1-[5-(4-ethoxybenzyl)-2-methoxy-4-methylphenyl]-1-thio-D-glucitol (hereinafter referred to as compound A) | 5 mg |
| Metformin hydrochloride | 500 mg |
| Microcrystalline cellulose | 70 mg |
| Hydroxypropyl cellulose | 25 mg |
| Carboxymethyl starch sodium | 30 mg |
| Magnesium stearate | 3 mg. |

(Preparation 2) Tablet

Tablets with a diameter of 7 mm were prepared; each tablet contained:

| | |
|---|---|
| Compound A | 5 mg |
| Pioglitazone hydrochloride | 16.53 mg |
| Microcrystalline cellulose | 48 mg |
| Lactose | 50 mg |
| Hydroxypropyl cellulose | 5 mg |
| Low-substitution hydroxypropyl cellulose | 14 mg |
| Magnesium stearate | 1 mg. |

(Preparation 3) Tablet

Tablets with a diameter of 7 mm were prepared; each tablet contained:

| | |
|---|---|
| Compound A | 5 mg |
| Glimepiride | 4 mg |
| Microcrystalline cellulose | 61 mg |
| Lactose | 50 mg |
| Hydroxypropyl cellulose | 5 mg |
| Low-substitution hydroxypropyl cellulose | 14 mg |
| Magnesium stearate | 1 mg. |

(Preparation 4) Tablet

Tablets with a diameter of 8 mm were prepared; each tablet contained:

| | |
|---|---|
| Compound A | 5 mg |
| Sitagliptin | 50 mg |
| Microcrystalline cellulose | 45 mg |
| Mannitol | 69 mg |
| Hydroxypropyl cellulose | 10 mg |
| Low-substitution hydroxypropyl cellulose | 20 mg |
| Magnesium stearate | 1 mg. |

Examples

On the following pages, the present invention is described in greater detail by means of examples, which are by no means intended to limit the scope of the present invention.

Test Example 1

<Test item>

Effects of chronic combination treatment of compound A and metformin hydrochloride in diabetic mice

<Test method>

Eight male db/db mice/group (11-week old; CLEA JAPAN, Inc.) were orally administered with repeated doses of compound A (3 mg/kg, once daily) and a biguanide, metformin hydrochloride (50 mg/kg or 150 mg/kg, twice daily; SIGMA-ALDRICH JAPAN K.K.), either alone or in combination, for 27 days. Eight db/m mice (11-week old; CLEA JAPAN, Inc.) were used as nondiabetic normal controls.

Before and 27th day after the start of repeated administration, blood was taken from the tail vein of each mouse

and centrifuged to collect hematocyte fractions. After hemolysis of the hematocyte fractions, glycated hemoglobin (GHb) values were measured by affinity column chromatography with an automated glycated hemoglobin analyzer (TOSOH CORPORATION). Starting at the 27th day of repeated administration, mice were fasted for 16 hours, thereafter, blood was taken from the tail vein of each mouse and centrifuged to collect plasma. Plasma glucose levels were measured by the mutarotase-GOD method with a glucose assay kit (Glucose C2 Test Wako; Wako Pure Chemical Industries, Ltd.) Further, before and 27th day after the start of repeated administration, blood was taken from the tail vein of each mouse under non-fasting conditions and centrifuged to collect plasma. Plasma insulin levels were measured by ELISA with an insulin assay kit (Levis: Mouse Insulin ELISA KIT (H-Type); SHIBAYAGI Co., Ltd.)

<Result 1>

The changes in GHb are expressed as the mean ± standard error in Table 1. The combination treatment of compound A (3 mg/kg) and metformin hydrochloride (300 mg/kg) markedly decreased the change in GHb, compared with each drug alone. A test by two-way ANOVA showed a significant interaction effect of compound A (3 mg/kg) and metformin hydrochloride (300 mg/kg) on the changes in GHb.

Table 1

| Group | Change in GHb (%) |
|---|---|
| Normal control | -0.05 ± 0.10 |
| Diabetic control | 1.20 ± 0.32 |
| Metformin hydrochloride, 100 mg/kg | 2.08 ± 0.33 |
| Metformin hydrochloride, 300 mg/kg | 1.70 ± 0.23 |
| Compound A, 3 mg/kg | 0.14 ± 0.39 |
| Compound A (3 mg/kg) + metformin hydrochloride (100 mg/kg) | 0.63 ± 0.27 |
| Compound A (3 mg/kg) + metformin hydrochloride (300 mg/kg) | -0.54 ± 0.15* |

Change in GHb (%) = GHb (%) after repeated administration − GHb (%) before start of repeated administration

*: P<0.05 for interaction effect of compound A (3 mg/kg) and metformin hydrochloride (300 mg/kg) (in two-way ANOVA)

<Result 2>

Fasting plasma glucose levels are expressed as the mean ± standard error in Table 2. The Student's t-test was used to detect the difference between two groups. The combination treatment of compound A (3 mg/kg) and metformin hydrochloride (300 mg/kg) markedly decreased the fasting plasma glucose level, compared with each drug alone.

Table 2

| Group | Fasting plasma glucose level (mg/dL) |
|---|---|
| Normal control | 76.2 ± 2.2 |
| Diabetic control | 416.2 ± 24.4 |
| Metformin hydrochloride, 100 mg/kg | 378.3 ± 33.4 |
| Metformin hydrochloride, 300 mg/kg | 254.0 ± 24.7 |
| Compound A, 3 mg/kg | 275.0 ± 28.7 |
| Compound A (3 mg/kg) + metformin hydrochloride (100 mg/kg) | 226.8 ± 18.3 §§ |

(continued)

| Group | Fasting plasma glucose level (mg/dL) |
|---|---|
| Compound A (3 mg/kg) + metformin hydrochloride (300 mg/kg) | 171.6 ± 13.5 ††, # |
| §§: P<0.01 vs. metformin hydrochloride (100 mg/kg) group<br>††: P<0.01 vs. compound A (3 mg/kg) group<br>#: P<0.05 vs. metformin hydrochloride (300 mg/kg) group. | |

<Result 3>

The changes in non-fasting plasma insulin (IRI) levels are expressed as the mean ± standard error in Table 3. The Student's t-test was used to detect the difference between two groups. The combination treatment of compound A (3 mg/kg) and metformin hydrochloride (300 mg/kg) markedly inhibited the decrease in plasma IRI levels, compared with each drug alone.

Table 3

| Group | Change in plasma IRI level (ng/mL) |
|---|---|
| Normal control | -1.23 ± 0.70 |
| Diabetic control | -9.15 ± 1.05 |
| Metformin hydrochloride, 100 mg/kg | -9.81 ± 0.91 |
| Metformin hydrochloride, 300 mg/kg | -7.36 ± 1.29 |
| Compound A, 3 mg/kg | -5.01 ± 2.11 |
| Compound A (3 mg/kg) + metformin hydrochloride (100 mg/kg) | -5.07 ± 1.50 § |
| Compound A (3 mg/kg) + metformin hydrochloride (300 mg/kg) | 1.88 ± 1.87 †, ## |
| Change in plasma IRI level (ng/mL) = plasma IRI level (ng/mL) after repeated administration - plasma IRI level (ng/mL) before start of repeated administration<br>§: P<0.05 vs. metformin hydrochloride (100 mg/kg) group<br>†: P<0.05 vs. compound A (3 mg/kg) group<br>##: P<0.01 vs. metformin hydrochloride (300 mg/kg) group. | |

Test Example 2

<Test item>

Effects of the combination treatment of compound A and glipizide in diabetic mice

<Test method>

After 3 weeks a high-fat diet (D12492 of Research Diets, Inc.) feeding, ICR mice (CHARLES RIVER LABORATORIES JAPAN, INC.) were intraperitoneally administered streptozocin (SIGMA-ALDRICH JAPAN K.K.; hereinafter

abbreviated as STZ) to induce diabetes. Under non-fasting conditions, the high-fat diet loaded mice with STZ-induced diabetes mellitus (15-week old) in groups each consisting of 11-12 mice were orally administered single doses of compound A (1 mg/kg) and an insulin secretagogue, glipizide (10 mg/kg; SIGMA-ALDRICH JAPAN K.K.), either alone or in combination. Blood was taken from the tail vein of each mouse at specified time intervals and plasma glucose levels were measured by GOD colorimetry with a self-testing glucose measuring instrument (Medisafe Mini GR-102; TERUMO CORPORATION, Japan).

<Results>

The areas under the plasma glucose levels curve for a period up to 8 hours after drug administration (⊿ plasma glucose AUC) are expressed as the mean ± standard error in Table 4. The Welch t-test was used to detect the difference between two groups. The combination treatment of compound A and glipizide markedly decreased ⊿ plasma glucose AUC, compared with each drug alone.

Table 4

| Group | ⊿ plasma glucose AUC (mg/dL x hr) |
|---|---|
| Diabetic control | 104.7 ± 137.4 |
| Compound A, 1 mg/kg | -310.3 ± 157.2 |
| Glipizide, 10 mg/kg | -222.3 ± 103.2 |
| Compound A (1 mg/kg) + glipizide (10 mg/kg) | -989.7 ± 232.8 †, ## |
| †: P<0.05 vs. compound A (1 mg/kg) group<br>##: P<0.01 vs. glipizide (10 mg/kg) group. | |

Test Example 3

<Test item>

Effects of the combination treatment of compound A and glimepiride in diabetic mice and normal mice

<Test method>

Under non-fasting conditions, 10 female KKAy mice/group (diabetic mice; 4-week old; CLEA Japan, Inc.) and 10 female C57BL mice/group (normal mice; 4-week old; CLEA Japan, Inc.) were orally administered with single doses of compound A (10 mg/kg) and an insulin secretagogue, glimepiride (0.5 mg/kg; SIGMA-ALDRICH JAPAN K.K.), either alone or in combination. Blood was taken from the tail vein of each mouse at specified time intervals and plasma glucose levels were measured by the GDH electrode method with a self-testing glucose measuring instrument (Glutest Neo Super; SANWA KAGAKU KENKYUSHO CO., LTD.)

<Results>

The areas under the plasma glucose levels curve for a period up to 3 hours after drug administration (⊿ plasma glucose AUC) are expressed as the mean ± standard error in Table 5 and 6. The two-way ANOVA was used to detect main effects and interaction effect of compound A and glimepiride. In KKAy mice (diabetic mice), the combination treatment of compound A and glimepiride markedly decreased ⊿ plasma glucose AUC, compared with each drug alone. On the other hand, the combination treatment of compound A and glimepiride decreased ⊿ plasma glucose AUC equally to treatment of glimepiride in normoglycemic C57BL mice (normal mice). The combined compound A with glimepiride exerted no more glucose lowering effect, compared with glimepiride alone. These results suggest that the combination treatment of compound A and glimepiride is expected to reduce the risk of hypoglycemia as a side effect.

Table 5 Effects of the combination treatment of compound A and glimepiride in KKAy mice

| Group | ⊿ plasma glucose AUC (mg/dL x hr) |
|---|---|
| Diabetic control | 0.1 ± 7.3 |
| Compound A, 10 mg/kg | -83.0 ± 14.6 |
| Glimepiride, 0.5 mg/kg | -118.6 ± 20.2 |
| Compound A (10 mg/kg) + glimepiride (0.5 mg/kg) | -151.5 ± 11.6 |
| Main effect of compound A (10 mg/kg): P<0.001<br>Main effect of glimepiride (0.5 mg/kg): P<0.0001<br>Interaction effect of compound A (10 mg/kg)and glimepiride (0.5 mg/kg): No significant difference (in two-way ANOVA). | |

Table 6 Effects of the combination treatment of compound A and glimepiride in C57BL mice

| Group | ⊿ plasma glucose AUC (mg/dL x hr) |
|---|---|
| Normal control | 16.3 ± 10.8 |
| Compound A, 10 mg/kg | -4.2 ± 8.5 |
| Glimepiride, 0.5 mg/kg | -104.0 ± 9.3 |
| Compound A (10 mg/kg) + glimepiride (0.5 mg/kg) | -97.1 ± 15.1 |
| Main effect of compound A (10 mg/kg): P=0.5516<br>Main effect of glimepiride (0.5 mg/kg): P<0.0001<br>Interaction effect of compound A (10 mg/kg) and glimepiride (0.5 mg/kg): No significant difference (in two-way ANOVA). | |

Test Example 4

<Test item>

Effects of chronic combination treatment of compound A and glimepiride in diabetic mice

<Test method>

Seven or eight female KKAy mice/group (4-week old; CLEA JAPAN, Inc.) were orally administered with repeated doses of compound A (0.03% mixed diet; ad libitum) and an insulin secretagogue, glimepiride (0.5 mg/kg, once daily; SIGMA-ALDRICH JAPAN K.K.), either alone or in combination, for 8 weeks.

After 4 weeks and 8 weeks, blood was taken from the tail vein of each mouse at one hour after the drug administration under non-fasting conditions. Plasma glucose levels were measured by the GDH electrode method with a self-testing glucose measuring instrument (Glutest Neo Super; SANWA KAGAKU KENKYUSHO CO., LTD.) In addition, body weights were measured both before and the days at 4th and 8th weeks after the start of repeated administration.

<Results>

The plasma glucose levels at the 4th and 8th weeks of repeated administration are expressed as the mean ± standard error in Table 7 and 8. The two-way ANOVA was used to detect main effects and interaction effect of compound A and glimepiride. Both at the 4th week and the 8th week of repeated administration, the combination treatment of compound A and glimepiride markedly decreased the plasma glucose level, compared with each drug alone.

The percent changes in body weights from the values before the start of repeated administration are expressed as the mean ± standard error in Table 9 and 10. The dunnett's test was used to detect the difference between two groups. Both at the 4th week and the 8th week of repeated administration, the glimepiride-treated mice tended to increase the body weights compared with diabetic control mice. On the other hand, the compound A-treated mice and the combination-treated mice significantly decreased body weights, compared with diabetic control mice.

These results show that the combination treatment with glimepiride and compound A markedly lowered the plasma glucose level and suppressed the body weight gain induced by glimepiride, suggesting the possibility of mitigating a side effect of glimepiride.

Table 7

| Group | Plasma glucose level at 4 wk of administration (mg/dL) |
|---|---|
| Diabetic control | 192.1 ± 3.5 |
| Mixed diet containing compound A, 0.03% | 154.8 ± 5.9 |
| Glimepiride, 0.5 mg/kg | 137.6 ± 13.3 |
| Mixed diet containing compound A (0.03%) + glimepiride (0.5 mg/kg) | 115.6 ± 16.1 |
| Data represented the plasma glucose level at one hour after drug administration.<br>Main effect of mixed diet containing compound A (0.03%): P<0.05<br>Main effect of glimepiride (0.5 mg/kg): P<0.001<br>Interaction effect of mixed diet containing compound A (0.03%) and glimepiride (0.5 mg/kg): No significant difference (in two-way ANOVA). | |

Table 8

| Group | Plasma glucose level at 8 wk of administration (mg/dL) |
|---|---|
| Diabetic control | 206.6 ± 9.8 |
| Mixed diet containing compound A, 0.03% | 168.6 ± 2.2 |
| Glimepiride, 0.5 mg/kg | 160.4 ± 8.2 |
| Mixed diet containing compound A (0.03%) + glimepiride (0.5 mg/kg) | 123.6 ± 6.2 |
| Data represented the plasma glucose level at one hour after drug administration.<br>Main effect of mixed diet containing compound A (0.03%): P<0.0001<br>Main effect of glimepiride (0.5 mg/kg): P<0.0001<br>Interaction effect of mixed diet containing compound A (0.03%) and glimepiride (0.5 mg/kg): No significant difference (in two-way ANOVA). | |

Table 9

| Group | Weight change (%) at 4 wk of administration |
|---|---|
| Diabetic control | 178.1 ± 4.3 |
| Mixed diet containing compound A, 0.03% | 138.0 ± 3.7*** |
| Glimepiride, 0.5 mg/kg | 189.9 ± 4.4 |
| Mixed diet containing compound A (0.03%) + glimepiride (0.5 mg/kg) | 144.1 ± 3.7*** |
| Weight change (%) represented the percent change in body weight from the value before the start of repeated administration.<br>***: P<0.001 vs. diabetic control group | |

Table 10

| Group | Weight change (%) at 8 wk of administration |
|---|---|
| Diabetic control | 218.9 ± 4.7 |
| Mixed diet containing compound A, 0.03% | 166.7 ± 6.0*** |

(continued)

| Group | Weight change (%) at 8 wk of administration |
|---|---|
| Glimepiride, 0.5 mg/kg | 232.5 ± 6.3 |
| Mixed diet containing compound A (0.03%) + glimepiride (0.5 mg/kg) | 166.7 ± 4.4*** |
| Weight change (%) represented the percent change in body weight from the value before the start of repeated administration.<br>***: P<0.001 vs. diabetic control group | |

Test Example 5

<Test item>

Effects of the combination treatment of compound A and glibenclamide in diabetic mice

<Test method>

After 3 weeks a high-fat diet (D12492 of Research Diets, Inc.) feeding, ICR mice (CHARLES RIVER LABORATORIES JAPAN, INC.) were intraperitoneally administered with streptozocin (SIGMA-ALDRICH JAPAN K.K.; hereinafter abbreviated as STZ) to induce diabetes. Under non-fasting conditions, the high-fat diet loaded mice with STZ-induced diabetes mellitus (12-week old) in groups each consisting of 10-12 mice were orally administered with single doses of compound A (1 mg/kg) and an insulin secretagogue, glibenclamide (10 mg/kg; SIGMA-ALDRICH JAPAN K.K.), either alone or in combination. Blood was taken from the tail vein of each mouse at specified time intervals and centrifuged to collect plasma. Plasma glucose levels were measured by mutarotase-GOD method with a glucose measuring kit (Glucose C2 Test Wako; Wako Pure Chemical Industries, Ltd.)

<Results>

The areas under the plasma glucose levels curve for a period up to 8 hours after drug administration (⊿ plasma glucose AUC) are expressed as the mean ± standard error in Table 11. The two-way ANOVA was used to detect main effects and interaction effect of compound A and glibenclamide. The combination treatment of compound A and glibenclamide markedly decreased ⊿ plasma glucose AUC, compared with each drug alone.

Table 11

| Group | ⊿ plasma glucose AUC (mg/dL x hr) |
|---|---|
| Diabetic control | 407.5 ± 67.4 |
| Compound A, 1 mg/kg | -130.1 ± 164.9 |
| Glibenciamide, 10 mg/kg | 140.3 ± 107.2 |
| Compound A (1 mg/kg) + glibenclamide (10 mg/kg) | -550.5 ± 167.4 |
| Main effect of compound A (1 mg/kg): P<0.0001<br>Main effect of glibenclamide (10 mg/kg): P<0.05<br>Interaction effect of compound A (1 mg/kg) and glibenclamide (10 mg/kg): No significant difference (in two-way ANOVA). | |

Test Example 6

<Test item>

Effects of chronic combination treatment of compound A and pioglitazone in diabetic mice

<Test method>

Eight male db/db mice/group (7-week old; CLEA JAPAN, Inc.) were orally administered with repeated doses of compound A (3 mg/kg, once daily) and an insulin sensitizer, pioglitazone (10 mg/kg, once daily; SIGMA-ALDRICH JAPAN K.K.), either alone or in combination, for 27 days. Eight db/m mice (7-week old; CLEA JAPAN, Inc.) were used as nondiabetic normal mice.

Before and 27th day after the start of repeated administration, blood was taken from the tail vein of each mouse and centrifuged to collect hematocyte fraction and plasma under non-fasting conditions. After hemolysis of the hematocyte fractions, glycated hemoglobin (GHb) values were measured by affinity column chromatography with an automated glycated hemoglobin analyzer (TOSOH CORPORATION). Plasma glucose levels were measured by the mutarotase-GOD method with a glucose assay kit (Glucose C2 Test Wako; Wako Pure Chemical Industries, Ltd.) Plasma insulin levels were measured by ELISA with insulin assay kits (Levis: Mouse Insulin ELISA KIT (H-type) and Mouse Insulin ELISA KIT (T-type); SHIBAYAGI Co., Ltd.)

<Result 1>

The changes in GHb are expressed as the mean ± standard error in Table 12. The two-way ANOVA was used to detect main effects and interaction effect of compound A and pioglitazone. The combination treatment of compound A and pioglitazone markedly decreased the change in GHb, compared with each drug alone.

Table 12

| Group | Change in GHb (%) |
|---|---|
| Diabetic control | 4.88 ± 0.22 |
| Compound A, 3 mg/kg | 3.15 ± 0.16 |
| Pioglitazone, 10 mg/kg | 3.04 ± 0.36 |
| Compound A (3 mg/kg) + pioglitazone (10 mg/kg) | 2.01 ± 0.14 |

Change in GHb (%) = GHb (%) after repeated administration - GHb (%) before repeated administration

Main effect of compound A (3 mg/kg): P<0.0001
Main effect of pioglitazone (10 mg/kg): P<0.0001
Interaction effect of compound A (3 mg/kg) and pioglitazone (10 mg/kg): No significant difference (in two-way ANOVA).

<Result 2>

Non-fasting plasma glucose levels are expressed as the mean ± standard error in Table 13. The two-way ANOVA was used to detect main effects and interaction effect of compound A and pioglitazone. The combination treatment of compound A and pioglitazone markedly decreased the non-fasting plasma glucose, compared with each drug alone.

Table 13

| Group | Non-fasting plasma glucose level (mg/dL) |
|---|---|
| Diabetic control | 795.1±12.9 |
| Compound A, 3 mg/kg | 607.9±21.6 |

(continued)

| Group | Non-fasting plasma glucose level (mg/dL) |
|---|---|
| Pioglitazone, 10 mg/kg | 555.2±56.9 |
| Compound A (3 mg/kg) + pioglitazone (10 mg/kg) | 424.9±31.1 |
| Main effect of compound A (3 mg/kg): P<0.0001<br>Main effect of pioglitazone (10 mg/kg): P<0.0001<br>Interaction effect of compound A (3 mg/kg) and pioglitazone (10 mg/kg): No significant difference (in two-way ANOVA). | |

<Result 3>

The change in non-fasting plasma insulin (IRI) level are expressed as the mean ± standard error in Table 14. The two-way ANOVA was used to detect main effects and interaction effect of compound A and pioglitazone. The combination treatment of compound A and pioglitazone markedly increased the change in non-fasting plasma IRI level. A test by two-way ANOVA showed a significant interaction effect of compound A and pioglitazone on the changes in non-fasting plasma IRI level. These results show that the combined compound A with pioglitazone improved superior glycemic control, compared with each drug alone, and probably contributed to synergistically suppressing the failure of pancreatic beta-cells by glucose toxicity.

Table 14

| Group | Change in plasma IRI level (ng/dL) |
|---|---|
| Diabetic control | -28.98 ± 2.88 |
| Compound A, 3 mg/kg | -27.81 ± 4.41 |
| Pioglitazone, 10 mg/kg | -43.29 ± 3.83 |
| Compound A (3 mg/kg) + pioglitazone (10 mg/kg) | 1.83 ± 10.02 |
| Main effect of compound A (3 mg/kg): P=0.2101<br>Main effect of pioglitazone (10 mg/kg): P<0.001<br>Interaction effect of compound A (3 mg/kg) and pioglitazone (10 mg/kg): P<0.01 (in two-way ANOVA). | |

Test Example 7

<Test item>

Effects of the combination treatment of compound A and insulin in diabetic rats

<Test method>

SD rats (7-week old; CHARLES RIVER LABORATORIES JAPAN, INC.) were intraperitoneally administered with streptozocin (SIGMA-ALDRICH JAPAN K.K.; hereinafter abbreviated as STZ) to induce diabetes. The rats with STZ-induced diabetes mellitus (8-week old) underwent subcutaneous implant of a slow-release insulin (one pellet of Linplant; LinShin Canada, Inc.) or sham operation. One week later, they were orally administered with a single dose of compound A (1 mg/kg) under non-fasting conditions. Blood was taken from the tail vein of each rat at specified time intervals and centrifuged to collect plasma. Plasma glucose levels were measured by mutarotase-GOD method with a glucose assay kit (Glucose C2 Test Wako; Wako Pure Chemical Industries, Ltd.)

<Results>

The areas under the plasma glucose levels curve for a period up to 8 hours after drug administration (plasma glucose AUC) are expressed as the mean ± standard error in Table 15. The two-way ANOVA was used to detect main effects and interaction effect of compound A and insulin. The combination treatment of compound A and insulin markedly decreased the plasma glucose AUC, compared with each drug alone.

Table 15

| Group | Plasma glucose AUC (mg/dL x hr) |
|---|---|
| Diabetic control | 4730.1 ± 127.6 |
| Compound A, 1 mg/kg | 3983.4 ± 194.5 |
| Slow-release insulin | 1532.6 ± 334.7 |
| Compound A (1 mg/kg) + slow-release insulin | 890.8 ± 86.7 |
| Main effect of compound A (1 mg/kg): P<0.01<br>Main effect of slow-release insulin: P<0.0001<br>Interaction effect of compound A (1 mg/kg) and slow-release insulin: No significant difference (in two-way ANOVA). | |

Test Example 8

<Test item>

Effects of the combination treatment of compound A and sitagliptin in diabetic rats

<Test method>

Eight male Zucker fatty rats/group (10-week old) were fasted and orally administered with single doses of compound A (1 mg/kg) and a DPP-IV inhibitor, sitagliptin (0.3 mg/kg), either alone or in combination. One hour after the administration, the rats were orally administered with glucose solutions (2 g/kg); blood was taken from the tail vein of each rat at specified time intervals and centrifuged to collect plasma. Plasma glucose levels were measured by mutarotase-GOD method with a glucose assay kit (Glucose C2 Test Wako; Wako Pure Chemical Industries, Ltd.)

<Results>

The areas under the plasma glucose levels curve for a period up to 120 minutes after glucose loading (Δ plasma glucose AUC) are expressed as the mean ± standard error in Table 16. The two-way ANOVA was used to detect main effects and interaction effect of compound A and sitagliptin. The Zucker fatty rat showed an increase in plasma glucose level after oral glucose loading, indicating impaired glucose tolerance. The combination treatment of compound A and sitagliptin markedly suppressed the increase in plasma glucose level after glucose loading, compared with each drug alone.

Table 16

| Group | Δ plasma glucose AUC (mg/dL x min) |
|---|---|
| Diabetic control | 17168.4 ± 1716.7 |
| Compound A, 1 mg/kg | 11239.9 ± 617.8 |
| Sitagliptin, 0.3 mg/kg | 13881.4 ± 1292.9 |
| Compound A (1 mg/kg) + sitagliptin (0.3 mg/kg) | 9385.2 ± 854.8 |
| Main effect of compound A (1 mg/kg): P<0.001<br>Main effect of sitagliptin (0.3 mg/kg): P<0.05<br>Interaction effect of compound A (1 mg/kg) and sitagliptin (0.3 mg/kg): No significant difference (in two-way ANOVA). | |

Test Example 9

<Test item>

Effects of the combination treatment of compound A and vildagliptin in diabetic rats

<Test method>

Eight male Zucker fatty rats (15-week old) were fasted and orally administered with single doses of compound A (1 mg/kg) and a DPP-IV inhibitor, vildagliptin (3 mg/kg), either alone or in combination. One hour after the administration, the rats were orally administered with glucose solutions (2 g/kg); blood was taken from the tail vein of each rat at specified time intervals and centrifuged to collect plasma. Plasma glucose levels were measured by mutarotase-GOD method with a glucose assay kit (Glucose C2 Test Wako; Wako Pure Chemical Industries, Ltd.)

<Results>

The areas under the plasma glucose levels curve for a period up to 120 minutes after glucose loading (Δ plasma glucose AUC) are expressed as the mean ± standard error in Table 17. The two-way ANOVA was used to detect main effects and interaction effect of compound A and vildagliptin. The Zucker fatty rat showed an increase in plasma glucose level after oral glucose loading, indicating impaired glucose tolerance. The combination treatment of compound A and vildagliptin markedly suppressed the increase in plasma glucose level after glucose loading, compared with each drug alone.

Table 17

| Group | Δ plasma glucose AUC (mg/dL x min) |
|---|---|
| Diabetic control | 19294.4±1788.0 |
| Compound A, 1 mg/kg | 11690.1±807.9 |
| Vildagliptin, 3 mg/kg | 15384.3±1740.7 |
| Compound A (1 mg/kg) + vildagliptin (3 mg/kg) | 8817.2±994.6 |
| Main effect of compound A (1 mg/kg): $P<0.0001$<br>Main effect of vildagliptin (3 mg/kg): $P<0.05$<br>Interaction effect of compound A (1 mg/kg) and vildagliptin (3 mg/kg): No significant difference (in two-way ANOVA). | |

Test Example 10

<Test item>

Effects of the combination treatment of compound A and voglibose in diabetic rats

<Test method>

Six male Zucker fatty rats (10-week old) were fasted and orally administered with single doses of compound A (1 mg/kg) and an α-glucosidase inhibitor, voglibose (0.1 mg/kg), either alone or in combination. One minute after the administration, the rats were orally administered with soluble starch solutions (2g/kg); blood was taken from the tail vein of each rat at specified time intervals and centrifuged to collect plasma. Plasma glucose levels were measured by mutarotase-GOD method with a glucose assay kit (Glucose C2 Test Wako; Wako Pure Chemical Industries, Ltd.)

<Results>

The areas under the plasma glucose levels curve for a period up to 120 minutes after glucose loading (Δ plasma glucose AUC) are expressed as the mean ± standard error in Table 18. The two-way ANOVA was used to detect main effects and interaction effect of compound A and voglibose. The Zucker fatty rat showed an increase in plasma glucose level after oral glucose loading, indicating impaired glucose tolerance. The combination treatment of compound A and

voglibose markedly suppressed the increase in plasma glucose level after glucose loading, compared with each drug alone.

Table 18

| Group | Δ plasma glucose AUC (mg/dL x min) |
|---|---|
| Diabetic control | 17548.4±1061.7 |
| Compound A, 1 mg/kg | 10940.4±952.7 |
| Voglibose, 0.1 mg/kg | 12395.5±543.0 |
| Compound A (1 mg/kg) + voglibose (0.1 mg/kg) | 7889.1±1197.0 |
| Main effect of compound A (1 mg/kg): P<0.0001<br>Main effect of voglibose (0.1 mg/kg): P<0.001<br>Interaction effect of compound A (1 mg/kg) and voglibose (0.1 mg/kg): No significant difference (in two-way ANOVA). | |

Test Example 11

<Test item>

Effects of the combination treatment of compound A and miglitol in diabetic rats

<Test method>

Six male Zucker fatty rats (9-week old) were fasted and orally administered with single doses of compound A (0.5 mg/kg) and an α-glucosidase inhibitor, miglitol (3 mg/kg), either alone or in combination. One minute after the administration, the rats were orally administered with soluble starch solutions (2 g/kg); blood was taken from the tail vein of each rat at specified time intervals and centrifuged to collect plasma. Plasma glucose levels were measured by mutarotase-GOD method with a glucose assay kit (Glucose C2 Test Wako; Wako Pure Chemical Industries, Ltd.)

<Results>

The areas under the plasma glucose levels curve for a period up to 120 minutes after glucose loading (Δ plasma glucose AUC) are expressed as the mean ± standard error in Table 19. The two-way ANOVA was used to detect main effects and interaction effect of compound A and miglitol. The Zucker fatty rat showed an increase in plasma glucose level after oral glucose loading, indicating impaired glucose tolerance. The combination treatment of compound A and miglitol markedly suppressed the increase in plasma glucose level after glucose loading, compared with each drug alone.

Table 19

| Group | Δ plasma glucose AUC (mg/dL x min) |
|---|---|
| Diabetic control | 15763.2±710.4 |
| Compound A, 0.5 mg/kg | 11655.9±1021.8 |
| Miglitol, 3 mg/kg | 8909.4±1390.0 |
| Compound A (0.5 mg/kg) + miglitol (3 mg/kg) | 7405.2±818.1 |
| Main effect of compound A (0.5 mg/kg): P<0.05<br>Main effect of miglitol (3 mg/kg): P<0.0001<br>Interaction effect of compound A (0.5 mg/kg) and miglitol (3 mg/kg): No significant difference (in two-way ANOVA). | |

Test Example 12

<Test item>

Effects of the combination treatment of compound A and acarbose in diabetic rats

<Test method>

Six male Zucker fatty rats (11-week old) were fasted and orally administered with single doses of compound A (1 mg/kg) and an α-glucosidase inhibitor, acarbose (1 mg/kg), either alone or in combination. One minute after the administration, the rats were orally administered with soluble starch solutions (2 g/kg); blood was taken from the tail vein of each rat at specified time intervals and centrifuged to collect plasma. Plasma glucose levels were measured by mutarotase-GOD method with a glucose assay kit (Glucose C2 Test Wako; Wako Pure Chemical Industries, Ltd.)

<Results>

The areas under the plasma glucose levels curve for a period up to 120 minutes after glucose loading (Δ plasma glucose AUC) are expressed as the mean ± standard error in Table 20. The two-way ANOVA was used to detect main effects and interaction effect of compound A and acarbose. The Zucker fatty rat showed an increase in plasma glucose level after oral glucose loading, indicating impaired glucose tolerance. The combination treatment of compound A and acarbose markedly suppressed the increase in plasma glucose level after glucose loading, compared with each drug alone.

Table 20

| Group | Δ plasma glucose AUC (mg/dL x min) |
|---|---|
| Diabetic control | 14464.7±1667.9 |
| Compound A, 1 mg/kg | 9861.6±1076.0 |
| Acarbose, 1 mg/kg | 10192.8±913.6 |
| Compound A (1 mg/kg) + acarbose (1 mg/kg) | 7510.3±776.6 |
| Main effect of compound A (1 mg/kg) group: P<0.01<br>Main effect of acarbose (1 mg/kg) group: P<0.01<br>Interaction effect of compound A (1 mg/kg) and acarbose (1 mg/kg): No significant difference (in two-way ANOVA). | |

Industrial Applicability

The present invention can provide superior pharmaceutical compositions for preventing or treating diabetes mellitus that exhibit an effective blood glucose lowering action in many diabetic patients and which yet cause fewer side effects. The present invention can also provide pharmaceutical compositions for preventing or treating various diabetic complications that result from hyperglycemia, such as diabetic retinopathy, diabetic nephropathy, diabetic neuropathy, cerebrovascular disorder, ischemic heart disease, and peripheral arterial disease.

## Claims

**1.** A pharmaceutical composition comprising a combination of

(A) (1S)-1,5-anhydro-1-[5-(4-ethoxybenzyl)-2-methoxy-4-methylphenyl]-1-thio-D-glucitol or a pharmaceutically acceptable salt thereof or a hydrate of the compound or the salt; and
(B) at least one member of the group consisting of dipeptidyl peptidase IV inhibitors, insulin sensitizers, insulins, α-glucosidase inhibitors, and GLP-1 mimetics.

**2.** The pharmaceutical composition according to claim 1, wherein (B) is a dipeptidyl peptidase IV inhibitor.

**3.** The pharmaceutical composition according to claim 2, wherein the dipeptidyl peptidase IV inhibitor is sitagliptin or vildagliptin.

**4.** The pharmaceutical composition according to claim 1, wherein the insulin sensitizer is pioglitazone.

**5.** The pharmaceutical composition according to claim 1, wherein the α-glucosidase inhibitor is voglibose, miglitol, or acarbose.

**6.** A combination of:

(A) (1S)-1,5-anhydro-1-[5-(4-ethoxybenzyl)-2-methoxy-4-methylphenyl]-1-thio-D-glucitol or a pharmaceutically acceptable salt thereof or a hydrate of the compound or the salt; and
(B) at least one member of the group consisting of dipeptidyl peptidase IV inhibitors, insulin sensitizers, insulins, α-glucosidase inhibitors, and GLP-1 mimetics,

for use in a method of preventing or treating diabetes mellitus, diseases associated with diabetes mellitus, or complications of diabetes mellitus, wherein (A) and (B) are administered to a patient in need simultaneously or separately.

**7.** The combination according to claim 6, wherein (B) is a dipeptidyl peptidase IV inhibitor.

**8.** The combination according to claim 7, wherein the dipeptidyl peptidase IV inhibitor is sitagliptin or vildagliptin.

**9.** The combination according to claim 6, wherein the insulin sensitizer is pioglitazone.

**10.** The combination according to claim 6, wherein the α-glucosidase inhibitor is voglibose, miglitol, or acarbose.

**11.** The combination according to any one of claims 6 to 10, wherein diabetes mellitus is type 2 diabetes.

**12.** The combination according to any one of claims 6 to 11, wherein the complication of diabetes mellitus is diabetic retinopathy, diabetic nephropathy, diabetic neuropathy, cerebrovascular disorder, ischemic cardiac disease, or peripheral arterial disease.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006073197 A1
- EP 1381361 B1
- WO 2009022007 A1
- WO 2009022010 A1
- WO 9608243 A1
- JP 2003520759 A


### Non-patent literature cited in the description


- Tonyobyougaku. Diabetology (Fundamentals and Clinical Applications). Nishimura Shoten, 2007, 949-954
- **EMIL A. WERNER ; JAMES BELL.** *J. Chem. Soc.,* 1922, vol. 121, 1790-1794